# EUROPEAN PATENT SPECIFICATION

(11) **EP 2 651 390 B1**
(45) Date of publication and mention of the grant of the patent: **27.05.2015**
(21) Application number: 11794519.6
(22) Date of filing: 15.12.2011
(51) Int. Cl.: A61K 9/46, A61K 47/02, A61K 9/50, A61K 31/437, A61K 33/10, A61K 9/00, A61K 9/20

(54) **ZOLPIDEM-BASED ORODISPERSIBLE PHARMACEUTICAL TABLET**
ZOLPIDEM-BASIERT PHARMAZEUTISCHE SCHMELZTABLETTE
COMPOSITION PHARMACEUTIQUE ORODISPERSIBLE COMPRENANT DU ZOLPIDEM

(30) Priority: 16.12.2010 FR 1060654
(43) Date of publication of application: 23.10.2013
(73) Proprietor: Sanofi, 75013 Paris (FR)
(72) Inventor: MARTINEZ, Annick, F-75013 Paris (FR); ROUGEOT, Olivier, F-75013 Paris (FR); YAGUCHI, Yoshikatsu, F-75013 Paris (FR)
(74) Representative: Taravella, Brigitte
(86) International application number: PCT/EP2011/072918
(87) International publication number: WO 2012/080399

(56) References cited:
- WO-A2-2009/071219
- FR-A1- 2 787 715
- US-A1- 2010 204 259

## Description

### Field of the invention

The invention relates to a zolpidem-based orodispersible pharmaceutical tablet formulation intended to prevent possible abuse of use of the tablet at the expense of a third party. It also relates to a process for the preparation of the said tablet and to the use thereof.

### Technological background and technical problem

Zolpidem is a hypnotic active principle of the imidazopyridine category. It is generally administered orally in the form of tablets or in other solid forms. Zolpidem acts rapidly. The pharmacokinetic and pharmacodynamic data show that zolpidem has a rapid absorption and a rapid triggering of the hypnotic action. Its bioavailability is 70% following oral administration and exhibits linear kinetics in therapeutic doses lying between 5 and 10 mg according to conventional formulations, the plasma concentration peak is reached between approximately 0.5 and 3 hours, and the elimination half-life is short, with a mean of 2.4 hours and a duration of action of up to 6 hours. However, there exists great variability between individuals in the bioavailability of a conventional immediate release formulation based on zolpidem. Thus, development of any new formulation comprising zolpidem is particularly problematic, it being necessary for the pharmacokinetic and pharmacodynamic data not to vary unacceptably with respect to the results previously obtained for the conventional formulations, such as for the immediate release formulations sold by the Applicant Company under the trade names Ambien®, Stilnox® and Myslee®.

There exists a need for a zolpidem-based pharmaceutical composition for rapid disintegration in the mouth which makes it possible in particular to swallow the composition without having to simultaneously drink and/or to be able to be more easily swallowed by people and in particular elderly people or people who frequently travel. As the hypnotic action of zolpidem is rapid, it is advisable to avoid any risk of falling immediately after taking the medicament. Thus, there exists a need to find a formulation which is easy to swallow immediately before going to bed, in particular without having to get up in order to swallow the formulation with a drink.

In addition, due to its hypnotic action, zolpidem might form the object of an abuse of use or of misuse. The administration of zolpidem might take place without the knowledge of a person, for example when the zolpidem-based formulation is introduced by a third party into a drink of this person, the said person then becoming submissive. The term "drug-facilitated crime" is thus used. There therefore also exists a need for a zolpidem-based formulation intended to prevent such an abusive use.

The Applicant Company has succeeded in developing a novel zolpidem-based formulation for rapid disintegration which can be easily detected in the event of misuse, while guaranteeing the patient a quality of treatment in accordance with his needs and/or comparable with a treating using conventional zolpidem formulations. The development of a formulation which makes possible both rapid disintegration and easy detection when misused is particularly complex in so far as the components introduced to counteract misuse can conflict with rapid disintegration of the said formulation.

It is thus an object of the present invention to provide a pharmaceutical composition for rapid disintegration in the mouth (or also known as orodispersible composition) comprising zolpidem which exhibits visual means which make it possible to easily detect the composition when the latter is introduced into a drink, optionally comprising alcohol, without the knowledge of the consumer of the drink.

Another object of the present invention is to provide a zolpidem-based orodispersible tablet exhibiting comparable dissolution and/or pharmacokinetic and/or pharmacological properties to conventional zolpidem-based formulations.

For reasons of simplicity, unless otherwise indicated, the term "zolpidem" in the description includes zolpidem or one of its pharmaceutically acceptable salts. The preferred zolpidem salt is zolpidem hemitartrate (or also known as zolpidem tartrate).

The orodispersible pharmaceutical tablet according to the invention makes it possible both to produce immediate release of zolpidem after oral administration in accordance with expectations and, if it is introduced into an optionally alcoholic drink, to generate visual means on contact with the latter. These visual means then make it possible to prevent the said drink from being administered to a person without his or her knowledge, the person being alerted visually by the presence of a foreign body (namely the tablet) in his or her drink.

Provision has already been made, in Patent Application WO 00/38649, for a pharmaceutical composition for oral administration intended to prevent the abuse of use of a pharmaceutical active principle at the expense of a third party. The visual means generated by the composition described correspond either to the floatation of the tablet, or to the formation of insoluble particles, optionally in combination with an effervescence, or to a combination of these visual means. However, the visual means consisting of the opacification of the drink as described below is neither described nor suggested. This visual means makes possible easy and rapid detection of the pharmaceutical composition in the drink, without this visual means troubling the patient who uses the pharmaceutical composition according to the recommended use.

### Summary of the invention

More specifically, a subject-matter of the present invention is an orodispersible pharmaceutical tablet, characterized in that it comprises zolpidem or one of its pharmaceutically acceptable salts, at least one disintegrating agent, at least one soluble agent of polyol type and components which, if the tablet is introduced into an aqueous drink optionally comprising alcohol, generate visual means corresponding to an opacification of the drink and to at least one other visual means chosen from effervescence, coloration, the formation of insoluble particles, the flotation of the tablet and a combination of these visual means, the components generating the said opacification of the drink comprising titanium dioxide particles.

According to a preferred embodiment of the invention, the components generating the said opacification of the drink consist of titanium dioxide particles.

Preferably, the tablet comprises at least 15 mg, preferably at least 20 mg, of titanium dioxide particles. Advantageously, the tablet comprises 15 mg of titanium dioxide particles.

According to another preferred embodiment, the tablet comprises at least one effervescence generator.

The effervescence generator is preferably composed solely of a carbon dioxide generator, the latter being chosen from a carbonate or a bicarbonate of an alkali metal, of an alkaline earth metal or of an amino acid, such as calcium carbonate, sodium bicarbonate, potassium bicarbonate, potassium carbonate, L-lysine carbonate, arginine carbonate, sodium sesquicarbonate and their mixture; advantageously, the carbon dioxide generator is calcium carbonate.

According to another specific embodiment, the tablet additionally comprises a lipophilic excipient chosen from glyceryl stearates, palmitate/stearates and behenates; hydrogenated vegetable oils and their derivatives; vegetable and animal waxes and their derivatives; hydrogenated castor oils and their derivatives; and cetyl esters and alcohols; preferably, the lipophilic excipient is glyceryl behenate.

The disintegrating agent is preferably chosen from sodium carboxymethylstarch, crosslinked sodium carboxymethylcellulose and crosslinked polyvinylpyrrolidone, and more advantageously crosslinked polyvinylpyrrolidone.

The amount of disintegrating agent preferably varies from 2 to 15% by weight, preferably from 4 to 12% by weight, with respect to the total weight of the tablet.

The soluble agent of polyol type is preferably chosen from mannitol, xylitol, sorbitol, maltitol and their mixture.

Preferably, the amount of soluble agent of polyol type is between 20 and 50% by weight, preferably between 30 and 45% by weight, with respect to total weight of the tablet.

According to a preferred embodiment, the zolpidem is coated with a coating agent based on a polymer which is soluble at pH 5 or less and which is permeable at a pH above 5.

Furthermore, the tablet preferably also comprises at least one hydrophilic excipient preferably chosen from cellulose, hydroxyethylcellulose, hydroxypropylcellulose, hydroxypropylmethylcellulose, carboxymethylcellulose or sodium carboxymethylcellulose derivatives; vegetable gums and their derivatives; alginic acid derivatives; polyethylene glycols and their derivatives; starches and their derivatives; silicas and their derivatives; polymethacrylates; and copolymers of acrylic and methacrylic acids.

Preferably, the zolpidem is in the form of granules, preferably with a mean particle diameter of between 100 and 300 µm, advantageously between 100 and 250 µm.

According to a specific embodiment, the tablet comprises (1) an internal phase comprising coated granules based on zolpidem and a coating agent based on a polymer which is soluble at pH 5 or less and which is permeable at a pH above 5, (2) an external phase comprising at least one disintegrating agent, at least one soluble agent of polyol type and components which, if the tablet is introduced into an aqueous drink which optionally comprises alcohol, generate visual means corresponding to an opacification of the drink and to at least one other visual means chosen from effervescence of the tablet, the flotation of the tablet, the formation of insoluble particles and the combination of these visual means, the components generating the said opacification of the drink being titanium dioxide particles.

Preferably, the internal phase comprises zolpidem granules coated with an amino-methacrylate copolymer.

In another preferred way, the disintegrating agent is crosslinked polyvinylpyrrolidone.

According to another preference, the soluble agent of polyol type is mannitol.

In another possible embodiment, the external phase comprises titanium dioxide particles, an agent which generates carbon dioxide and at least one lipophilic excipient.

More preferably, the external phase comprises titanium dioxide particles, glycerol behenate and optionally calcium carbonate, and is preferably devoid of pharmaceutically acceptable acid compound, when calcium carbonate is present.

The tablet preferably comprises from 1 to 5% by weight of zolpidem, such as, for example, 2.5, 5 or 10 mg per tablet, at least 20 mg, preferably at least 25 mg, of titanium dioxide particles per tablet, from 2 to 15%, preferably from 4 to 12%, of disintegrating agent and from 20 to 50%, preferably from 30 to 45%, of soluble agent of polyol type, the percentages being expressed with respect to the total weight of the tablet.

According to another alternative form, this tablet additionally comprises from 5 to 25% by weight, preferably from 10 to 20% by weight and more particularly 15% by weight of effervescence generator and from 0.05 to 15%, preferably from 0.1 to 6% and advantageously from 0.2 to 5% (inclusive) of lipophilic excipients.

Generally, the zolpidem salt used is zolpidem hemitartrate.

The invention also relates to a tablet according to the invention for oral use in a method for the treatment of insomnia, in particular occasional, transient or chronic insomnia.

The tablet according to the invention is particularly advantageous since it makes it possible to reveal its antimisuse properties when it is introduced into a drink but not to reveal them in the mouth.

The tablet according to the invention also exhibits the advantage of having a release and an action of the active principle which are not detrimentally affected by the orodispersible formulation and the presence of components intended to be revealed during a possible misuse.

In the context of the present invention, the pharmaceutical tablet is for immediate release.

### Brief Description of the Figures

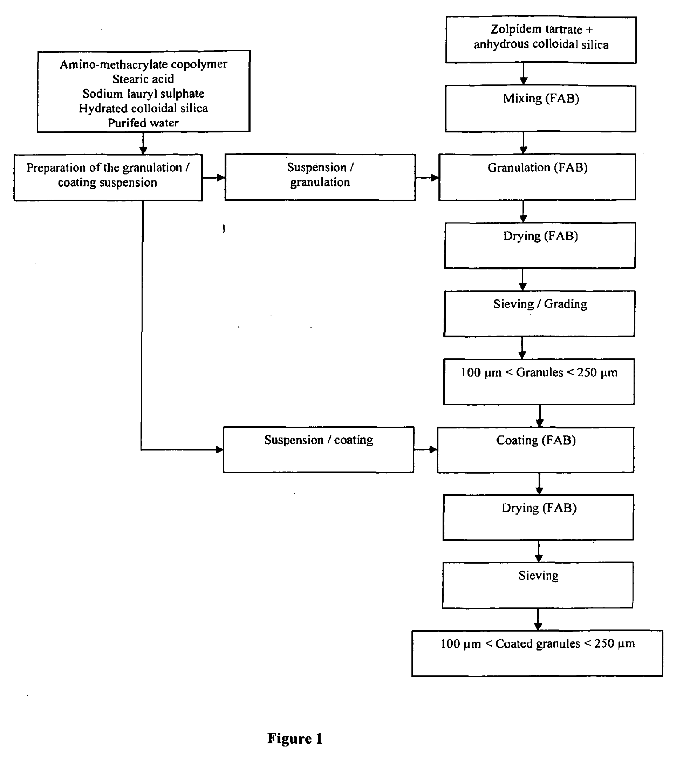

The main stages of the preparation of coated granules according to specific form of the invention are presented in Figure 1 (FAB means Fluidized Air Bed).

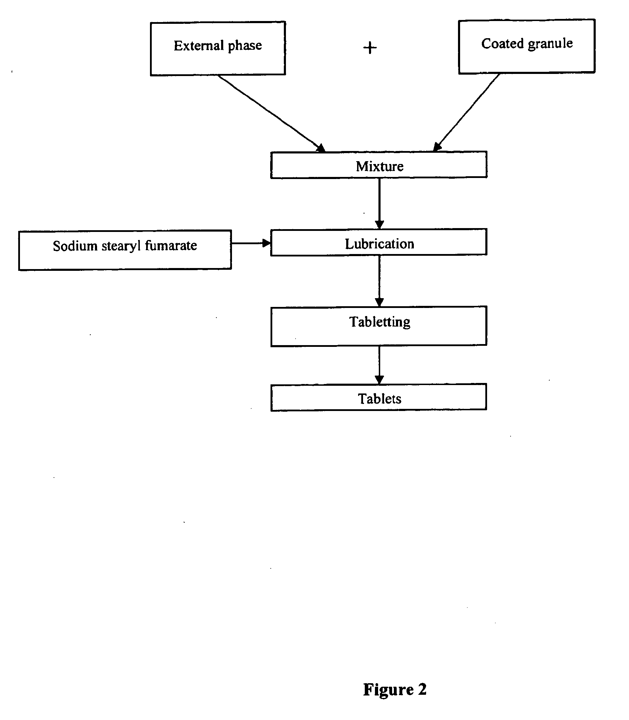

The main final stages of the preparation of tablets according to a specific form of the invention are presented in Figure 2.

### Detailed Description of the Invention

In the context of the present invention, the term "visual means" is understood to mean any element which indicates the presence of the tablet according to the invention in an aqueous drink optionally comprising alcohol and which can take the forms, for example, of an opacity, of an effervescence, of a dye, of flotation of the tablet at the surface of the drink or of formation of insoluble particles at the surface of the drink, on the edges of the receptacle, in the drink and/or on the bottom of the receptacle.

The aqueous drink can be transparent, translucent or opaque, can be coloured or colourless and can optionally comprise alcohol. It can in particular correspond to coffee, tea, wine, spirits, champagne, sake, any fizzy drink, such as a fizzy drink of cola type, optionally with the addition of alcohol, any other carbonated beverage, with or without alcohol, or any cocktail or liquid mixture based on fruit juice, on alcohols, and the like. The tablet according to the invention is particularly suitable for transparent or translucent drinks.

The tablets according to the present invention have a size acceptable for conventional oral administration. Thus, preference is given to tablets with a weight of less than 800 mg, preferably of less than 500 mg and more particularly of less than 400 mg (such as, for example, tablets having a weight of 100 mg, 150 mg, 200 mg, 250 mg or 400 mg).

According to the invention, the tablet comprises at least one disintegrating agent and at least one diluting agent of polyol type which make possible the rapid disintegration thereof in the mouth.

The term "rapid disintegration in the mouth" is understood to mean a tablet which breaks up in less than 3 minutes, preferably in less than 30 seconds (for example in 15, 20 or 30 seconds). Breaking up can be measured according to the test described in section 2.9.1 of the European Pharmacopoeia, using a device of Sotax DT3 type (preferably, the test is carried out without a disc).

Mention may in particular be made, among disintegrating agents, of sodium carboxymethylstarch (molecular weight of from 500 to 1000 kDa), such as the product sold by Avebe under the trade name Primojel® or the product sold by JRS Pharma under the trade name Explotab®, crosslinked sodium carboxymethylcellulose, also denoted by the term sodium croscarmellose, or crosslinked polyvinylpyrrolidone or crospovidone, such as the product sold by BASF under the trade name Kolidon® CL. Crosslinked polyvinylpyrrolidone is very particularly preferred.

The amount of the disintegrating agent in the tablet can vary within wide limits, in particular as a function of the other components present in the tablet and of their respective amounts. To give an order of magnitude and advantageously, the amount of the disintegrating agent can vary from 2 to 15% (inclusive) by weight, preferably from 4 to 12% (inclusive) by weight and advantageously from 7 to 10% (inclusive) (for example: 10%) by weight, with respect to the total weight of the tablet.

The tablet according to the invention can additionally comprise at least one soluble agent of polyol (or sugar alcohol) type. A polyol (or sugar alcohol) is a hydrogenated form of sugar in which the carbonyl group (i.e., aldehyde or ketone) has been reduced to give a primary or secondary hydroxyl group. In particular, the diluting soluble agent is chosen from a polyol of less than 13 carbon atoms, preferably of from 5 to 12 carbon atoms (inclusive), this polyol preferably being chosen from the group consisting of mannitol, xylitol, sorbitol, maltitol and their mixture. Preferably, mannitol is used. The said soluble agent is used in the form of granules, of powders or of a mixture of granules and powders. The granules generally exhibit a mean diameter of from 100 to 500 micrometres; in the form of a powder, the soluble agent exhibits a mean particle diameter generally of less than 100 micrometres, in particular between 10 and 90 micrometres.

The amount of the soluble agent in the tablet can vary within wide limits, in particular as a function of the other components present in the tablet and of their respective amounts. To give an order of magnitude and advantageously, the amount of the diluting soluble agent having binding properties can vary from 20 to 50% (inclusive) by weight, preferably from 30 to 45% (inclusive) (for example: 31, 32, 33, 34, 35, 42 and 43%) by weight, with respect to the total weight of the tablet.

At least one of the components of the tablet according to the invention generates an opacification of the drink when the tablet is introduced into an aqueous drink optionally comprising alcohol. This opacification of the drink is in particular advantageously obtained by the presence of titanium dioxide (TiO₂) in the tablet.

The TiO₂ is more specifically white in colour. It is provided in the form of particles. These particles are generally insoluble in water and organic solvents. When the tablet breaks up in a drink, the TiO₂ particles present in the tablet then form a suspension in the drink which is particularly visible by the consumer of the drink.

The TiO₂ particles are in the form of an odourless and tasteless white powder. The particles measure approximately 0.3 µm and generally at most 1 µm and often form aggregates of approximately 100 µm. The titanium dioxide is sold in particular by Brenntag under the name Titanium Dioxide 3328 ^{®}. The TiO₂ particles preferably exhibit a specific surface of greater than 10 m²/g and advantageously of less than 200 m²/g. More specifically, the specific surface of the TiO₂ particles is greater than 10 and less than 15 m²/g.

More particularly, the tablet according to the invention comprises at least 15 mg, or at least 20 mg, of TiO₂ particles (such as, for example, 15 or 30 or 60 mg per tablet). This minimal amount makes possible detection in a receptacle, such as a glass or a cup, containing a drink (the volume of the drink generally being at most 250 ml, preferably 200 or 75 ml, and even 4 cl). In the tablet, the amount of titanium dioxide particles is preferably between 8 and 30% by weight, with respect to the total weight of the tablet. Advantageously, the amount of titanium dioxide particles is preferably between 10 and 25% by weight, with respect to the total weight of the tablet (such as, for example, 12, 15 or 20%).

According to a specific form of the invention, the visual means correspond at least to opacification and to effervescence. According to this form, the tablet according to the invention preferably comprises zolpidem, titanium dioxide particles and at least one effervescence generator, preferably an effervescence generator composed solely of an agent which generates carbon dioxide.

One of the other visual means is thus effervescence (when the tablet is introduced into an aqueous drink optionally comprising alcohol). Effervescence is generally obtained by means of an effervescence generator, such as described below. The flotation of the tablet (or of its disintegrated particles) can optionally be provided by the effervescence.

The effervescence generator can be provided either in the form of an effervescent pair, composed of an agent which generates carbon dioxide and a pharmaceutically acceptable acid compound, or composed solely of an agent which generates carbon dioxide, the acidity of the drink making possible effervescence.

The agent which generates carbon dioxide is usually a carbonate or a bicarbonate of an alkali metal, of an alkaline earth metal or of an amino acid. Mention may be made, for example, as the agent which generates carbon dioxide, of calcium carbonate, sodium bicarbonate, potassium bicarbonate, potassium carbonate, L-lysine carbonate, arginine carbonate, sodium sesquicarbonate or their mixture. Preference is given, as the agent which generates carbon dioxide, to calcium carbonate.

The pharmaceutically acceptable acid compound is an acid anhydride, a monocarboxylic acid, a polycarboxylic acid or a polycarboxylic acid partial salt. The pharmaceutically acceptable acid compound can more particularly be chosen from citric acid, tartaric acid, ascorbic acid, fumaric acid, nicotinic acid, acetylsalicylic acid, malic acid, adipic acid, succinic acid, glutaric anhydride, citric anhydride, maleic acid, malonic acid, monosodium citrate and succinic anhydride.

Preference is given, as effervescence-generating pair, to the effervescent pair composed of calcium carbonate and anhydrous citric acid.

The agent which generates carbon dioxide can be composed of a mixture of several abovementioned agents which generate carbon dioxide.

In the effervescence generator, the content of acid compound is generally chosen so that the ratio of the number of moles of acid compound to the number of moles of carbon dioxide generator is between 1 and 2.

According to a preferred form, the effervescence generator is composed solely of an agent which generates carbon dioxide. According to this form, the tablet according to the invention is devoid of pharmaceutically acceptable acid compound. According to this form, the agent which generates carbon dioxide can be one of those mentioned above and is preferably calcium carbonate.

This is because it has been found that the majority of the drinks used in the context of misuse are acidic (pH of less than 7) and that they thus make it possible for effervescence to take place solely in the presence of agent which generates carbon dioxide. In addition, this form exhibits the advantage of not having the phenomenon of the effervescence which happens in the mouth during the oral administration of the tablet, the mouth generally being at a non-acid pH.

In this context, the amount of effervescence-generating agent is preferably between 5 and 25% by weight, advantageously between 10 and 20% (inclusive) by weight and more particularly 15% by weight, with respect to the total weight of the tablet.

The tablet can release visible insoluble particles even if the tablet does not float or does not immediately float.

According to a specific form, in order to provide for the formation of insoluble particles, other than titanium dioxide, visible at the surface of the drink and/or on the walls of the receptacle, the tablet can comprise one or more lipophilic excipients.

Mention may be made, among the lipophilic excipients, of glyceryl stearates, palmitate/stearates and behenates; hydrogenated vegetable oils and their derivatives; vegetable and animal waxes and their derivatives; hydrogenated castor oils and their derivatives; and cetyl esters and alcohols. Preference is given, among lipophilic excipients, to glyceryl behenate.

The amount of lipophilic excipients in the tablet can vary to a large extent, especially as a function of the active principle, of the other components present in the tablet and of their respective amounts. To give an order of magnitude, and advantageously, the amount of lipophilic excipients can vary from 0.05 to 15% (inclusive) by weight, preferably from 0.1 to 6% by weight and advantageously from 0.2 to 5% (inclusive) (for example: 0.25, 3 or 4%) by weight, with respect to the total weight of the tablet.

The tablet, in the liquid or at its surface, can break up, in particular under the action of effervescence. As break up occurs, the formation of agglomerates is observed, which agglomerates float and possibly become stuck to the walls of the receptacle and/or become suspended in the liquid.

According to a specific form of the invention, the visual means correspond at least to opacification, effervescence and to the formation of insoluble particles. According to this form, the tablet according to the invention comprises zolpidem, titanium dioxide particles, at least one effervescence generator, preferably an effervescence generator composed solely of an agent which generates carbon dioxide, and at least one lipophilic excipient.

The insoluble particles can thus optionally be obtained by the presence of a lipophilic excipient in the tablet according to the invention.

The tablet can additionally exhibit viscosity properties which appear on contact with any drink. This characteristic makes possible trapping of the gas possibly produced by the effervescence which is also reflected by a swelling of the tablet. The fall in density generated then makes it possible to provide for the maintenance of the tablet at the surface of the drink and thus makes possible flotation of the tablet or of its disintegrated particles. Such a viscosity can be obtained by virtue of one or more gelifiable compounds.

The gelifiable compound can comprise one or more hydrophilic excipients which bring about the swelling of the tablet and optionally the trapping of the gas formed by the effervescence generator. The gelifiable compound can correspond to a mixture of lipophilic and hydrophilic excipients.

The tablet according to the invention in addition optionally comprises at least one hydrophilic excipient. Mention may be made, among hydrophilic excipients, of derivatives of cellulose, hydroxyethylcellulose, hydroxypropylcellulose (molecular weight of from 50 to 1250 kDa), hydropropylmethylcellulose (molecular weight of from 10 to 1500 kDa), carboxymethylcellulose and sodium carboxymethylcellulose; vegetable gums and their derivatives; alginic acid derivatives; polyethylene glycols and their derivatives; starches and their derivatives; silicas and their derivatives; polymethacrylates; and copolymers of acrylic and methacrylic acids.

At least one of the constituents of the gelifiable compound, either the hydrophilic excipient or the lipophilic excipient, can be chosen so as to have little solubility in alcohol.

Thus, the tablet according to the invention, in particular the tablet, which has little solubility in an alcoholic liquid or drink with a degree of greater than 45, gives the liquid or the drink the appearance of a suspension of insoluble viscous particles.

Finally, another visual means, such as a colorant, can advantageously be added to the tablet according to the invention. This is of particular use when the drink is a translucent drink or fruit juice. This colorant can thus colour the drink. The colorant is generally neither black nor white.

Mention may be made, among the colorants which can be used in the tablet according to the invention, of indigotin, cochineal red, orange yellow S, allura red AC, iron oxides, cucurmin, riboflavin, tartrazine, quinoline yellow, azorubin, amaranth, carmines, erythrosine, red 2G, patent blue V, brilliant blue FCF, chlorophylls, cupric complexes of chlorophylls, green S, caramels, brilliant black BN, vegetable charcoal, brown FK and HT, carotenes, annatto extracts, paprika extracts, lycopene, lutein, canthaxanthin, beetroot red, anthocyanins, litholrubin BK or any other colorant suitable for consumption.

Finally, the technical preparation of the tablets can also lead to the introduction of tabletting agents:
- lubricating agents, such as magnesium stearate, stearic acid, glyceryl monostearate, polyethylene glycols having a molecular weight of from 400 to 7 000 000, hydrogenated castor oil, glyceryl behenate, mono-, bi- or trisubstituted glycerides, or sodium stearyl fumarate, sold under the name of Pruv® by JRS Pharma;
- flow agents, such as colloidal silica or any other silica, for example the product sold by Degussa under the trade name Aerosil;
- binders, such as starch, buffers, absorbents, diluents, such as lactose, and any other pharmaceutically acceptable additive.

According to an alternative form of the invention, the zolpidem in the tablet according to the invention is coated, encapsulated and/or pretreated, in order to mask the unpleasant taste of the active principle. This is because active principles, such as zolpidem and in particular zolpidem tartrate, have a bitter taste which may have to be masked. Various technologies, such as in particular granulation, coating, optionally in a fluidized air bed, and the like, can be employed in the context of the masking of the taste of active principles where the taste is unpleasant to the patient. The compounds for masking the taste which are used in the context of these technologies can be polymers: namely cellulose derivatives (HPC, HPMC, EC), methacrylic acid derivatives (Eudragit range) and polyvinyl acetate derivatives. It is also possible to envisage microencapsulation by a supercritical CO₂ phase process or by a simple or complex coacervation method. The same abovementioned masking polymers can be used, along with other excipients of chitosan, alginate or gelatin type. "Hot" (spray drying) or "cold" (spraying cooling) atomization processes can also be employed to mask the taste of an active principle. The "spray cooling" technology is described in WO2004/058137 and consists in melting a fatty matrix and in incorporating the active principle therein. The latter is either dissolved or dispersed in the fatty phase, composed of fatty acids, or esters of fatty acids, or fatty alcohols, or waxes, or mixture thereof. The molten mixture is subsequently brought, via a peristaltic pump, up to a twin-fluid nozzle where it is atomized into more or less fine droplets (depending on the parameters applied). Finally, the droplets are cooled by a stream of cold air in the atomization chamber and converted into solid granules comprising the active principle dispersed throughout the fatty matrix. It is also possible to envisage the use of a hot melt coating process. A hot melt coating process is in particular that which consists in preparing an active principle composition comprising (a) a granulated centre composed of active principle grains agglomerated in the presence of binder, preferably a hydrophilic agent chosen from hydrophilic polymers or heat-fusible agents (such as cellulose derivatives (hydroxypropylcellulose), povidone (polyvinylpyrrolidone), sucrose, gums, starches, gelatin or macrogols (polyethylene glycols)), and (b) a coating layer for the said granulated centre composed of a fatty matrix (such as stearic acid, palmitic acid or myristic acid, pure or as mixtures, and/or their corresponding fatty alcohols). This technology is described in particular in Patent Application FR1053034, filed on 21 April 2010 by the Applicant Company.

These technologies make it possible to obtain tablets with correct taste masking while providing for dissolution of the active principle in an acidic medium which is not slowed down.

According to a specific embodiment of the invention, the zolpidem is coated with a coating agent based on a polymer which is soluble at pH 5 or less and which is permeable at a pH above 5. Mention may in particular be made, as such, of amino-methacrylate copolymers of E type sold by Evonik® under the trade mark Eudragit® E, such as Eudragit® E100 (in the form of granules) or Eudragit® EPO (in the form of powders). Preference is very particularly given to the amino-methacrylate copolymer in the form of powders (such as Eudragit® EPO).

The zolpidem is generally provided in the form of crystals having a mean diameter of approximately 100 µm. Advantageously, in particular if it is coated, the zolpidem is granulated beforehand in order to preferably exhibit a mean particle diameter of between 100 and 300 µm, advantageously between 100 and 250 µm. The granulation can be carried out, for example, according to a conventional technique by mixing the different compounds, in order to obtain a homogeneous powder, wetting with an alcoholic, aqueous or aqueous/alcoholic solution and then drying, in a fluidized air bed, until a predetermined residual moisture percentage is obtained. The granules are subsequently preferably sieved, in order to recover the granules exhibiting a mean particle diameter of between 100 and 300 µm, advantageously between 100 and 250 µm. The solution advantageously comprises a binder, such as, for example, polyvinylpyrrolidone (molecular weight of from 28 to 1500 kDa), hydroxypropylmethylcellulose (or hypromellose), an amino-methacrylate copolymer of E type as described above or any other pharmaceutically acceptable binder. It is preferable to use the amino-methacrylate copolymer.

The coating of the zolpidem is carried out by conventional techniques, such as, for example, by mixing the granules of active principle as obtained above with a solution comprising the coating agent as defined above, followed by drying in a fluidized air bed. The solution thus comprises the coating agent, composed of at least one polymer which is soluble at pH 5 or less and which is permeable at a pH above 5; in addition it comprises at least one surface-active agent, at least one plasticizer, at least one anti-blocking agent, at least one solvent and preferably a mixture of these components. Preferably, the amount of dry matter of the solution to be deposited on the granules is between 50 and 99% by weight, advantageously from 60 to 70% (inclusive) by weight, with respect to the total weight of the coated granule. Preferably, the amount of the coating agent in the solution is between 50 and 70% by weight, more particularly between 60 and 65% by weight, with respect to the total weight of the coated granule.

The tablet according to the present invention can be obtained by any conventional tabletting technology known to a person skilled in the art by mixing powders and/or granulating using, for example, rotary presses. Thus, the components of the tablet according to the invention are mixed with one another either in solution or in the form of powders and/or granules, in order subsequently to be optionally dried, then tabletted. The mixture necessary for the tabletting can be obtained by dry granulation or direct mixing of the constituents. The constituents are thus those specified above, in particular zolpidem, and in particular in the form of granules, optionally coated, encapsulated and/or pretreated, as described above, the disintegrating agent, the soluble agent of polyol type, titanium dioxide particles, one or more other component(s) against misuse, and optionally other constituents, such as those specified above in the text, such as, for example, lubricating agents, flow agents and/or binders.

A tablet can take a cylindrical, lenticular, spheroidal, ovoidal or other shape which makes possible easy administration and easy swallowing.

The tablet according to the invention is preferably devoid of film coating at its external surface.

The amounts of zolpidem necessary are the same as those which are usually administered. By way of example, the amount of zolpidem hemitartrate is between 2 and 10 mg, such as, for example, 2.5, 5 and 10 mg, per tablet.

A specific embodiment of the orodispersible tablet according to the invention is a tablet comprising (1) an internal phase comprising coated granules based on zolpidem and a coating agent based on a polymer which is soluble at pH 5 or less and which is permeable at a pH above 5, (2) an external phase comprising at least one disintegrating agent, at least one soluble agent of polyol type and components which, if the tablet is introduced into an aqueous drink which optionally comprises alcohol, generate visual means corresponding to an opacification of the drink and to at least one other visual means chosen from effervescence of the tablet, the flotation of the tablet, the formation of insoluble particles and the combination of these visual means, the components generating the said opacification of the drink being titanium dioxide particles.

According to a specific form, the internal phase of the above tablet comprises zolpidem granules coated with an amino-methacrylate copolymer; preferably, in the external phase, the disintegrating agent is crosslinked polyvinylpyrrolidone and/or the soluble agent of polyol type is mannitol.

Preferably, the external phase comprises titanium dioxide particles, an agent which generates carbon dioxide, in particular calcium carbonate, and at least one lipophilic excipient, in particular glyceryl behenate. Advantageously, the external phase or more generally the tablet is devoid of pharmaceutically acceptable acid compound.

According to a specific form, the tablet according to the invention comprises an external phase comprising titanium dioxide particles, calcium carbonate and glyceryl behenate which is devoid of pharmaceutically acceptable acid compound.

This specific embodiment is particularly advantageous for the reasons described above, namely the tablet according to the invention is capable, if it is introduced into a drink optionally comprising alcohol, of generating visual means on contact with the latter, it reveals its antimisuse properties when it is introduced into a drink but it does not reveal them in the mouth, and the release and the action of the zolpidem, when it is administered orally, are in accordance with expectations. The release and the action of the active principle are not detrimentally affected by the presence, in the tablet, of the constituents intended to be revealed during a possible misuse.

According to a specific form, the tablet according to the invention generally comprises from 1 to 5% by weight of zolpidem, such as, for example, 2.5, 5 or 10 mg per tablet, comprises at least 15 mg of titanium dioxide particles per tablet, comprises from 2 to 15% (inclusive), preferably from 4 to 12% (inclusive), of disintegrating agent and comprises from 20 to 50%, preferably from 30 to 45%, of soluble agent of polyol type, the percentages being expressed with respect to the total weight of the tablet. Advantageously, the tablet additionally comprises from 5 to 25% by weight, preferably from 10 to 20% by weight, of effervescence generator and from 0.05 to 15% (inclusive), preferably from 0.1 to 6%, of lipophilic excipients.

Another subject-matter of the present invention is a method for treating insomnia, in particular occasional, transient or chronic insomnia, comprising the oral administration to a subject requiring it of at least one zolpidem-based orodispersible tablet according to the invention.

The present invention also relates to the zolpidem-based orodispersible tablet according to the invention for oral use in a method for the treatment of insomnia, in particular occasional, transient or chronic insomnia.

Finally, the present invention relates to the tablet according to the invention for use in a therapeutic treatment method, in particular a method for the treatment of insomnia and more specifically occasional, transient or chronic insomnia, intended to prevent abuse of use of the tablet at the expense of a third party.

The subject is more specifically a mammal and very particularly a human being.

The following examples illustrate the present invention without wishing, however, to limit the scope thereof.

### Examples

### Example 1

### Orodispersible zolpidem tartrate tablets, dosage 10 mg

### Manufacturing process (see Figures 1 and 2):

Zolpidem tartrate is mixed with anhydrous colloidal silica in a fluidized bed. The mixture is subsequently granulated in a fluidized bed using a suspension of amino-methacrylate copolymer. This suspension is preprepared by dispersing amino-methacrylate copolymer, sodium lauryl sulphate, stearic acid and hydrated colloidal silica in purified water, according to the recommendations of the supplier of amino-methacrylate copolymer.

**Table 1**

| **Composition** | **Unit formula (mg)** | **Percentage formula (%)** |
|---|---|---|
| Zolpidem tartrate | 10.00 | 4.00 |
| Anhydrous colloidal silica (Aerosil® 200, sold by Evonik Degussa GmbH^{®}) | 0.20 | 0.08 |
| Amino-methacrylate copolymer (Eudragit^{®} EPO, sold by Evonik Degussa GmbH^{®}) | 13.90 | 5.56 |
| Sodium lauryl sulphate (sold by Cognis^{®}) | 1.38 | 0.55 |
| Stearic acid (Stearic Acid 2236^{®}, sold by Mallinckrodt Baker^{®}) | 2.06 | 0.83 |
| Hydrated colloidal silica (Syloïd^{®} FP 244, sold by GRACE Davison^{®}) | 4.82 | 1.93 |
| Mannitol (Pearlitol^{®} 160C, sold by Roquette^{®}, and/or Parteck M200, sold by Merck^{®}) | 95.14 | 34.06 |
| Crosslinked polyvinylpyrrolidone (Kollidon^{®} CL, sold bv ISP^{®}) | 25.00 | 10.00 |
| Aspartame (sold by Ajinomoto Sweeteners Europe SAS) | 3.75 | 1.50 |
| Sodium stearyl fumarate (Pruv^{®}, sold by JRS Pharma^{®}) | 3.75 | 1.50 |
| Titanium dioxide (Titanium Dioxide 3328^{®}, sold by Cognis^{®}) | 30.00 | 12.00 |
| Microcrystalline cellulose (Avicel^{®} PH 101, sold by FMC Biopolymer^{®}) | 25.00 | 10.00 |
| Glyceryl behenate (Compritol 888 ATO^{®}, sold by Gattefossé^{®}) | 7.50 | 3.00 |
| Calcium carbonate (Hubercal^{®} 150, sold by Huber^{®}) | 37.50 | 15.00 |
| Total | 250.00 | 100.00 |

### The zolpidem tartrate/anhydrous colloidal silica mixture is granulated by spraying, by top spray, the suspension based on amino-methacrylate copolymer and is then dried.

The dry granule obtained is graded and sieved, so as to select the particles having a size of between 100 µm and 250 µm.

The granules selected are subsequently coated with this same suspension of amino-methacrylate copolymer in a fluidized bed equipped with a Wurster column (bottom spray spraying process). Once the amount of suspension has been deposited, the granule is dried and then sieved, so as to retain only the particles having a size of between 100 µm and 250 µm.

The coated granule obtained is subsequently mixed in a tumbler mixer with the external phase comprising mannitol, crosslinked polyvinylpyrrolidone, aspartame, titanium dioxide particles, glyceryl behenate and calcium carbonate. Finally, the mixture is lubricated using sodium stearyl fumarate in the tumbler mixer.

The mannitol used is either in the form of a powder (Pearlitol 160C), of granules (Parteck M200) or of a mixture of powder and granules; in particular, the ratios of powder/granules percentages by weight varying from 60/40-100/0 were tested.

The final mixture obtained is tabletted on a rotary tabletting press.

The composition is described in detail in Table 1.

Breaking up was measured according to the test described in section 2.9.1 of the European Pharmacopoeia, using a device of Sotax DT3 type. The test was carried out without the disc. The result obtained is 13 s.

The antimisuse effectiveness was evaluated by introducing a tablet into different drinks. The results obtained are presented in the following Table 2.

**Table 2**

| **Drink evaluated** | **Appearance of the drink after introduction of the tablet** | **Visual detection of the tablet** |
|---|---|---|
| Water | Cloudy liquid with white suspended particles | Yes |
| Orange juice | White particles floating at the surface of the drink | Yes |
| Fizzy drink of cola type | Significant effervescence of the fizzy drink with presence of white particles in the foam | Yes |
| Wine | Presence of floating white particles at the surface of the liquid | Yes |
| Whisky | Cloudiness of the liquid with presence of white suspended particles | Yes |

### Example 2

### Orodispersible zolpidem tartrate tablets, dosage 5 mg

The orodispersible zolpidem tartrate tablets, dosed at 5 mg, are manufactured according to the same manufacturing process as that described in Example 1.

The composition is described in detail in the following Table 3.

**Table 3**

| **Composition** | **Unit formula (mg)** | **Percentage formula (%)** |
|---|---|---|
| Zolpidem tartrate | 5.00 | 2.50 |
| Anhydrous colloidal silica (Aerosil^{®} 200, sold by Evonik Degussa GmbH^{®}) | 0.10 | 0.05 |
| Amino-methacrylate copolymer (Eudragit^{®} EPO, sold by Evonik Degussa GmbH^{®}) | 6.93 | 3.47 |
| Sodium lauryl sulphate (sold by Cognis^{®}) | 0.68 | 0.34 |
| Stearic acid (Stearic Acid 2236^{®}, sold by Mallinckrodt Baker^{®}) | 1.03 | 0.51 |
| Hydrated colloidal silica (Syloid^{®} FP 244, sold by GRACE Davison^{®}) | 2.40 | 1.20 |
| Mannitol (Pearlitol^{®} 160C, sold by Roquette^{®}, and/or Parteck M200, sold by Merck^{®}) | 71.86 | 35.92 |
| Crosslinked polyvinylpyrrolidone (Kollidon^{®} CL, sold by ISP^{®}) | 20.00 | 10.00 |
| Aspartame (sold by Ajinomoto sweeteners Europe SAS) | 3.00 | 1.50 |
| Sodium stearyl fumarate (Pruv^{®}, sold by JRS Pharma^{®}) | 3.00 | 1.50 |
| Titanium dioxide (Titanium Dioxide 3328^{®}, sold by Cognis^{®}) | 30.00 | 15.00 |
| Microcrystalline cellulose (Avicel^{®} PH 101, sold by FMC Biopolymer^{®}) | 20.00 | 10.00 |
| Glyceryl behenate (Compritol 888 ATO^{®}, sold by Gattefossé^{®}) | 6.00 | 3.00 |
| Calcium carbonate (Hubercal^{®} 150, sold by Huber^{®}) | 30.00 | 15.00 |
| Total | 200.00 | 100.00 |

Breaking up was measured according to the test described in section 2.9.1 of the European Pharmacopoeia using a device of Sotax DT3 type. The test was carried out with a disc. The result obtained is 14 s.

### Example 3

### Orodispersible zolpidem tartrate tablets, dosage 2.5 mg

The orodispersible zolpidem tartrate tablets, dosed at 2.5 mg, are manufactured according to the same manufacturing process as that described in Example 1.

The composition is described in detail in the following Table 4.

**Table 4**

| **Composition** | **Unit formula (mg)** | **Percentage formula (%)** |
|---|---|---|
| Zolpidem tartrate | 2.50 | 1.67 |
| Anhydrous colloidal silica (Aerosil^{®} 200, sold by Evonik Degussa GmbH^{®}) | 0.05 | 0.03 |
| Amino-methacrylate copolymer(Eudragit^{®} EPO, sold bv Evonik Degussa GmbH^{®}) | 3.47 | 2.32 |
| Sodium lauryl sulphate (sold by Cognis^{®}) | 0.34 | 0.23 |
| Stearic acid (Stearic Acid 2236^{®}, sold by Mallinckrodt Baker^{®}) | 0.52 | 0.34 |
| Hydrated colloidal silica (Syloïd^{®} FP 244, sold by GRACE Davison^{®}) | 1.20 | 0.80 |
| Mannitol (Pearlitol^{®} 160C, sold by Roquette^{®}, and/or Parteck M200, sold by Merck^{®}) | 50.40 | 33.6 |
| Crosslinked polyvinylpyrrolidone (Kollidon^{®} CL, sold by ISP^{®}) | 15.00 | 10.00 |
| Aspartame (sold by Ajinomoto sweeteners Europe SAS) | 2.25 | 1.50 |
| Sodium stearyl fumarate (Pruv^{®}, sold by JRS Pharma^{®}) | 2.25 | 1.50 |
| Titanium dioxide (Titanium Dioxide 3328^{®}, sold by Cognis^{®}) | 30.00 | 15,00 |
| Microcrystalline cellulose (Avicel^{®} PH 101, sold bv FMC Biopolymer^{®}) | 15.00 | 10.00 |
| Glyceryl behenate (Compritol 888 ATO^{®}, sold bv Gattefossé^{®}) | 4.50 | 3.00 |
| Calcium carbonate (Hubercal^{®} 150, sold by Huber^{®}) | 22.5 | 15.00 |
| Total | 150.00 | 100.00 |

Breaking up was measured according to the test described in section 2.9.1 of the European Pharmacopoeia using a device of Sotax DT3 type. The test was carried out without a disc. The result obtained is 14 s.

The tablets according to Examples 1-3 exhibit comparable dissolution curves in a 0.01 N HCl medium (900 ml, paddle at 50 rpm (revolutions per minute)) to the immediate release tablets with the trade names Myslee® 5 mg or 10 mg and Silnox® 10 mg.

### Example 4

Orodispersible zolpidem tartrate tablets, dosages 2.5 mg, 5.0 mg and 10.0 mg The orodispersible zolpidem tartrate tablets thus dosed are manufactured according to the same manufacturing process as that described in Example 1.

The compositions are described in detail in the following Tables 5, 6 and 7.

**Table 5**

| **Composition** | **Unit formula (mg)** | **Percentage formula (%)** |
|---|---|---|
| Zolpidem tartrate | 2.50 | 1.67 |
| Anhydrous colloidal silica (Aerosil® 200, sold by Evonik Degussa GmbH^{®}) | 0.05 | 0.05 |
| Amino-methacrylate copolymer (Eudragit^{®} EPO, sold by Evonik Degussa GmbH^{®}) | 2.84 | 2.84 |
| Sodium lauryl sulphate (sold by Cognis^{®}) | 0.29 | 0.29 |
| Stearic acid (Stearic Acid 2236^{®}, sold by Mallinckrodt Baker^{®}) | 0.42 | 0.42 |
| Hydrated colloidal silica (Syloid^{®} FP 244, sold by Grace Davison^{®}) | 0.98 | 0.98 |
| Zolpiden tartrate granule subtotal | 7.08 | 7.08 |
| Mannitol powder (Pearlitol^{®} 160C, sold by Roquette^{®}) | 25.60 | 25.60 |
| Mannitol granules (Parteck M200, sold by Merck^{®}) | 17.07 | 17.14 |
| Crosslinked polyvinylpyrrolidone (Kollidon^{®} CL, sold by ISP^{®}) | 7.00 | 7.00 |
| Aspartame (sold by Ajinomoto Sweeteners Europe SAS) | 1.50 | 1.50 |
| Sodium stearyl fumarate (Pruv^{®}, sold by JRS Pharma^{®}) | 1.50 | 1.50 |
| Titanium Dioxide (Titanium Dioxide 3328^{®}, sold by Cognis^{®}) | 15.00 | 15.00 |
| Microcrystalline cellulose (Avicel^{®} PH 101, sold by FMC Biopolymer^{®}) | 10.00 | 10.00 |
| Glyceryl behenate (Compritol 888 ATO^{®}, sold by Gattefossé^{®}) | 0.25 | 0.25 |
| Calcium carbonate (Hubercal^{®} 150, sold by Huber^{®}) | 15.00 | 15.00 |
| Total | 100.00 | 100.00 |

**Table 6**

| **Composition** | **Unit formula (mg)** | **Percentage formula (%)** |
|---|---|---|
| Zolpidem tartrate | 5.00 | 2.50 |
| Anhydrous colloidal silica (Aerosil® 200, sold by Evonik Degussa GmbH^{®}) | 0.10 | 0.05 |
| Amino-methacrylate copolymer (Eudragit^{®} EPO, sold by Evonik Degussa GmbH^{®}) | 5.69 | 2.85 |
| Sodium lauryl sulphate (sold by Cognis^{®}) | 0.57 | 0.29 |
| Stearic acid (Stearic Acid 2236^{®}, sold by Mallinckrodt Baker^{®}) | 0.84 | 0.42 |
| Hydrated colloidal silica (Syloid^{®} FP 244, sold by Grace Davison^{®}) | 1.97 | 0.99 |
| Zolpiden tartrate granule subtotal | 14.17 | 7.09 |
| Mannitol powder (Pearlitol^{®} 160C, sold by Roquette^{®}) | 51.20 | 25.60 |
| Mannitol granules (Parteck M200, sold by Merck^{®}) | 34.13 | 17.07 |
| Crosslinked polyvinylpyrrolidone (Kollidon^{®} CL, sold by ISP^{®}) | 14.00 | 7.00 |
| Aspartame (sold by Ajinomoto Sweeteners Europe SAS) | 3.00 | 1.50 |
| Sodium stearyl fumarate (Pruv^{®}, sold by JRS Pharma^{®}) | 3.00 | 1.50 |
| Titanium Dioxide (Titanium Dioxide 3328^{®}, sold by Cognis^{®}) | 30.00 | 15.00 |
| Microcrystalline cellulose (Avicel^{®} PH 101, sold by FMC Biopolymer^{®}) | 20.00 | 10.00 |
| Glyceryl behenate (Compritol 888 ATO^{®}, sold by Gattefossé^{®}) | 0.50 | 0.25 |
| Calcium carbonate (Hubercal^{®} 150, sold by Huber^{®}) | 30.00 | 15.00 |
| Total | 200.00 | 100.00 |

**Table 7**

| **Composition** | **Unit formula (mg)** | **Percentage formula (%)** |
|---|---|---|
| Zolpidem tartrate | 10.00 | 2.50 |
| Anhydrous colloidal silica (Aerosil® 200, sold by Evonik Degussa GmbH^{®}) | 0.20 | 0.05 |
| Amino-methacrylate copolymer (Eudragit^{®} EPO, sold by Evonik Degussa GmbH^{®}) | 11.37 | 2.84 |
| Sodium lauryl sulphate (sold by Cognis^{®}) | 1.14 | 0.29 |
| Stearic acid (Stearic Acid 2236^{®}, sold by Mallinckrodt Baker^{®}) | 1.69 | 0.42 |
| Hydrated colloidal silica (Syloid^{®} FP 244, sold by Grace Davison^{®}) | 3.93 | 0.98 |
| Zolpiden tartrate granule subtotal | 28.33 | 7.08 |
| Mannitol powder (Pearlitol^{®} 160C, sold by Roquette^{®}) | 102.40- | 25.60 |
| Mannitol granules (Parteck M200, sold by Merck^{®}) | 68.26 | 17.07 |
| Crosslinked polyvinylpyrrolidone (Kollidon^{®} CL, sold by ISP^{®}) | 28.00 | 7.00 |
| Aspartame (sold by Ajinomoto Sweeteners Europe SAS) | 6.00 | 1.50 |
| Sodium stearyl fumarate (Pruv^{®}, sold by JRS Pharma^{®}) | 6.00 | 1.50 |
| Titanium Dioxide (Titanium Dioxide 3328^{®}, sold by Cognis^{®}) | 60.00 | 15.00 |
| Microcrystalline cellulose (Avicel^{®} PH 101, sold by FMC Biopolymer^{®}) | 40.00 | 10.00 |
| Glyceryl behenate (Compritol 888 ATO^{®}, sold by Gattefossé^{®}) | 1.00 | 0.25 |
| Calcium carbonate (Hubercal^{®} 150, sold by Huber^{®}) | 60.00 | 15.00 |
| Total | 400.00 | 100.00 |

Breaking up was measured for these tablets according to the test described in section 2.9.1 of the European Pharmacopoeia, using a device of Sotax DT3 type. The test was carried out without the disc. The results obtained are 17 s approximately for these three compositions.

The antimisuse effectiveness was evaluated by introducing these tablets into different drinks. The results obtained are similar for the three compositions. The results thus obtained for the three compositions are presented in the following Table 8.

**Table 8**

| **Drink evaluated** | **Appearance of the drink after introduction of the tablet** | **Visual detection of the tablet** |
|---|---|---|
| Water | Cloudy liquid with blue suspended particles | Yes |
| Orange juice | Change in colour of the orange juice to green | Yes |
| Fizzy drink of cola type | Significant effervescence of the fizzy drink with presence of blue particles in the foam | Yes |
| Wine | Presence of white particles floating at the surface of the liquid | Yes |
| Whisky | Cloudiness of the liquid with greenish coloration | Yes |

## Claims

1. Orodispersible pharmaceutical tablet, **characterized in that** it comprises zolpidem or one of its pharmaceutically acceptable salts, at least one disintegrating agent, at least one soluble agent of polyol type and components which, if the tablet is introduced into an aqueous drink optionally comprising alcohol, generate visual means corresponding to an opacification of the drink and to at least one other visual means chosen from effervescence, the formation of insoluble particles, the flotation of the tablet and a combination of these visual means, the components generating the said opacification of the drink comprising titanium dioxide particles.

2. Tablet according to Claim 1, **characterized in that** the components generating the opacification of the drink consist of titanium dioxide particles.

3. Tablet according to either of Claims 1 and 2, **characterized in that** it comprises at least 15 mg of titanium dioxide particles.

4. Tablet according to one of Claims 1 to 3, **characterized in that** it comprises at least one effervescence generator; advantageously, the said generator is composed solely of a carbon dioxide generator, preferably chosen from a carbonate or a bicarbonate of an alkali metal, of an alkaline earth metal or of an amino acid, such as calcium carbonate, sodium bicarbonate, potassium bicarbonate, potassium carbonate, L-lysine carbonate, arginine carbonate, sodium sesquicarbonate and their mixture; advantageously, the carbon dioxide generator is calcium carbonate.

5. Tablet according to any one of Claims 1 to 4, **characterized in that** it additionally comprises a lipophilic excipient chosen from glyceryl stearates, palmitate/stearates and behenates; hydrogenated vegetable oils and their derivatives; vegetable and animal waxes and their derivatives; hydrogenated castor oils and their derivatives; and cetyl esters and alcohols; preferably, the lipophilic excipient is glyceryl behenate.

6. Tablet according to any one of the preceding claims, **characterized in that** the disintegrating agent is chosen from sodium carboxymethylstarch, crosslinked sodium carboxymethylcellulose and crosslinked polyvinylpyrrolidone, and more advantageously crosslinked polyvinylpyrrolidone.

7. Tablet according to any one of the preceding claims, **characterized in that** the soluble agent of polyol type is chosen from mannitol, xylitol, sorbitol, maltitol and their mixture; preferably, the soluble agent of polyol type is mannitol.

8. Tablet according to any one of the preceding claims, **characterized in that** the amount of the soluble agent of polyol type is between 20 and 50% by weight, preferably between 30 and 45% by weight, with respect to the total weight of the tablet.

9. Tablet according to any one of the preceding claims, **characterized in that** the zolpidem is coated with a coating agent based on a polymer which is soluble at pH 5 or less and which is permeable at pH above 5; in particular, the coating agent is an amino-methacrylate copolymer.

10. Tablet according to any one of the preceding claims, **characterized in that** it additionally comprises at least one hydrophilic excipient preferably chosen from cellulose, hydroxyethylcellulose, hydroxypropylcellulose, hydroxypropylmethylcellulose, carboxymethylcellulose or sodium carboxymethylcellulose derivatives; vegetable gums and their derivatives; alginic acid derivatives; polyethylene glycols and their derivatives; starches and their derivatives; silicas and their derivatives; polymethacrylates; and copolymers of acrylic and methacrylic acids.

11. Tablet according to any one of Claims 1 to 10, **characterized in that** it comprises (1) an internal phase comprising coated granules based on zolpidem and a coating agent based on a polymer which is soluble at pH 5 or less and which is permeable at a pH above 5, (2) an external phase comprising at least one disintegrating agent, at least one soluble agent of polyol type and components which, if the tablet is introduced into an aqueous drink which optionally comprises alcohol, generate visual means corresponding to an opacification of the drink and to at least one other visual means chosen from effervescence of the tablet, the flotation of the tablet, the formation of insoluble particles and the combination of these visual means, the components generating the said opacification of the drink being titanium dioxide particles.

12. Tablet according to Claim 11, **characterized in that** the external phase comprises titanium dioxide particles, an agent which generates carbon dioxide and at least one lipophilic excipient.

13. Tablet according to Claim 12, **characterized in that** the external phase comprises titanium dioxide particles, calcium carbonate and glycerol behenate and is preferably devoid of pharmaceutically acceptable acid compound.

14. Tablet according to any one of the preceding claims, **characterized in that** it comprises from 1 to 5% by weight of zolpidem, such as, for example, 2.5, 5 or 10 mg per tablet, at least 15 mg of titanium dioxide particles per tablet, from 2 to 15%, preferably from 4 to 12%, of disintegrating agent and from 20 to 50%, preferably from 30 to 45%, of soluble agent of polyol type, and preferably it additionally comprises from 5 to 25% by weight, preferably from 10 to 20% by weight, of effervescence generator and from 0.05 to 15%, preferably from 0.1 to 6%, of lipophilic excipients, the percentages being expressed with respect to the total weight of the tablet.

15. Tablet according to any one of the preceding claims for oral use in a method for the treatment of insomnia, in particular occasional, transient or chronic insomnia.

## Patentansprüche

1. Pharmazeutische Schmelztablette, **dadurch gekennzeichnet, dass** sie Zolpidem oder eines seiner pharmazeutisch verträglichen Salze, wenigstens ein Sprengmittel, wenigstens ein lösliches Mittel vom Polyol-Typ sowie Komponenten umfasst, die bei Einführen der Tablette in ein wässriges Getränk, das gegebenenfalls Alkohol umfasst, visuelle Mittel erzeugen, die einer Trübung des Getränks entsprechen, und wenigstens ein weiteres visuelles Mittel ausgewählt aus Brausen, der Entstehung unlöslicher Partikel, dem Aufschwimmen der Tablette und einer Kombination dieser visuellen Mittel, wobei die Komponenten, die die Trübung des Getränks erzeugen, Titandioxid-Partikel umfassen.

2. Tablette gemäß Anspruch 1, **dadurch gekennzeichnet, dass** die Komponenten, die die Trübung des Getränks erzeugen, aus Titandioxid-Partikeln bestehen.

3. Tablette gemäß einem der Ansprüche 1 und 2, **dadurch gekennzeichnet, dass** sie wenigstens 15 mg an Titandioxid-Partikeln umfasst.

4. Tablette gemäß einem der Ansprüche 1 bis 3, **dadurch gekennzeichnet, dass** sie wenigstens einen Brausegenerator umfasst; wobei der Generator vorteilhaft ausschließlich aus einem Kohlendioxidgenerator besteht, vorzugsweise ausgewählt aus einem Carbonat oder einem Bicarbonat eines Alkalimetalls, eines Erdalkalimetalls oder einer Aminosäure, wie z. B. Calciumcarbonat, Natriumbicarbonat, Kaliumbicarbonat, Kaliumcarbonat, L-Lysincarbonat, Arginincarbonat, Natriumsesquicarbonat und deren Gemisch; wobei der Kohlendioxidgenerator vorteilhaft Calciumcarbonat ist.

5. Tablette gemäß einem der Ansprüche 1 bis 4, **dadurch gekennzeichnet, dass** sie zusätzlich einen lipophilen Hilfsstoff ausgewählt aus Glycerylstearaten, -palmitat/stearaten und -behenaten; hydrierten Pflanzenölen und deren Derivaten; Pflanzen- und Tierwachsen und deren Derivaten; hydrierten Castorölen und deren Derivaten; und Cetylestern und -alkoholen umfasst; wobei der lipophile Hilfsstoff vorzugsweise Glycerylbehenat ist.

6. Tablette gemäß einem der vorstehenden Ansprüche, **dadurch gekennzeichnet, dass** das Sprengmittel ausgewählt ist aus Natriumcarboxymethylstärke, vernetzter Natriumcarboxymethylcellulose und vernetztem Polyvinylpyrrolidon, vorteilhafter vernetztem Polyvinylpyrrolidon.

7. Tablette gemäß einem der vorstehenden Ansprüche, **dadurch gekennzeichnet, dass** das lösliche Mittel vom Polyol-Typ ausgewählt ist aus Mannit, Xylit, Sorbit, Maltit und deren Gemisch; wobei das lösliche Mittel vom Polyol-Typ vorzugsweise Mannit ist.

8. Tablette gemäß einem der vorstehenden Ansprüche, **dadurch gekennzeichnet, dass** die Menge des löslichen Mittels vom Polyol-Typ zwischen 20 und 50 Gew.-%, vorzugsweise zwischen 30 und 45 Gew.-%, bezogen auf das Gesamtgewicht der Tablette beträgt.

9. Tablette gemäß einem der vorstehenden Ansprüche, **dadurch gekennzeichnet, dass** das Zolpidem mit einem Beschichtungsmittel auf der Grundlage eines Polymers, das bei einem pH-Wert von 5 oder weniger löslich ist und das bei einem pH-Wert von über 5 durchlässig ist, beschichtet ist; wobei das Beschichtungsmittel insbesondere ein Amino-Methylacrylat-Copolymer ist.

10. Tablette gemäß einem der vorstehenden Ansprüche, **dadurch gekennzeichnet, dass** sie zusätzlich wenigstens einen hydrophilen Hilfsstoff ausgewählt aus Cellulose-, Hydroxyethylcellulose-, Hydroxypropylcellulose-, Hydroxypropylmethylcellulose-, Carboxymethylcellulose- und Natriumcarboxymethylcellulosederivaten; Pflanzengummis und deren Derivaten; Alginsäurederivaten; Polyethylenglycolen und deren Derivaten; Stärken und deren Derivaten; Silicas und deren Derivaten; Polymethacrylaten; und Copolymeren von Acryl- und Methacrylsäuren umfasst.

11. Tablette gemäß einem der Ansprüche 1 bis 10, **dadurch gekennzeichnet, dass** sie (1) eine innere Phase, umfassend beschichtete Granulatkörner auf der Grundlage von Zolpidem und einem Beschichtungsmittel auf der Grundlage eines Polymers, das bei einem pH-Wert von 5 oder weniger löslich ist und das bei einem pH-Wert von über 5 durchlässig ist, (2) eine äußere Phase, umfassend wenigstens ein Sprengmittel, wenigstens ein lösliches Mittel vom Polyol-Typ und Komponenten, die bei Einführen der Tablette in ein wässriges Getränk, das gegebenenfalls Alkohol umfasst, visuelle Mittel erzeugen, die einer Trübung des Getränks entsprechen, und wenigstens ein weiteres visuelles Mittel ausgewählt aus Brausen der Tablette, dem Aufschwimmen der Tablette, der Entstehung unlöslicher Partikel und einer Kombination dieser visuellen Mittel, wobei die Komponenten, die die Trübung des Getränks erzeugen, Titandioxid-Partikel sind, umfasst.

12. Tablette gemäß Anspruch 11, **dadurch gekennzeichnet, dass** die äußere Phase Titandioxid-Partikel, ein Mittel, das Kohlendioxid erzeugt, und wenigstens einen lipophilen Hilfsstoff umfasst.

13. Tablette gemäß Anspruch 12, **dadurch gekennzeichnet, dass** die äußere Phase Titandioxid-Partikel, Calciumcarbonat und Glycerolbehenat umfasst und vorzugsweise frei von pharmazeutisch verträglichen Säureverbindungen ist.

14. Tablette gemäß einem der vorstehenden Ansprüche, **dadurch gekennzeichnet, dass** sie von 1 bis 5 Gew.-% an Zolpidem, wie z. B. 2,5, 5 oder 10 mg pro Tablette, wenigstens 15 mg an Titandioxid-Partikeln pro Tablette, von 2 bis 15 %, vorzugsweise von 4 bis 12 %, an Sprengmittel und von 20 bis 50 %, vorzugsweise von 30 bis 45 %, an löslichem Mittel vom Polyol-Typ umfasst und vorzugsweise zusätzlich von 5 bis 25 Gew.-%, vorzugsweise von 10 bis 20 Gew.-%, an Brausegenerator und von 0,05 bis 15 %, vorzugsweise von 0,1 bis 6 %, an lipophilen Hilfsstoffen umfasst, wobei die Prozentwerte bezogen auf das Gesamtgewicht der Tablette ausgedrückt sind.

15. Tablette gemäß einem der vorstehenden Ansprüche für die orale Verwendung bei einem Verfahren zum Behandeln von Schlaflosigkeit, insbesondere von gelegentlicher, vorübergehender oder chronischer Schlaflosigkeit.

## Revendications

1. Comprimé pharmaceutique orodispersible, **caractérisé en ce qu'**il comprend du zolpidem ou un de ses sels pharmaceutiquement acceptables, au moins un agent délitant, au moins un agent soluble de type polyol et des composants qui, si le comprimé est introduit dans une boisson aqueuse comprenant facultativement de l'alcool, génèrent un moyen visuel correspondant à une opacification de la boisson et à au moins un autre moyen visuel choisi parmi l'effervescence, la formation de particules insolubles, le flottage du comprimé et une combinaison de ces moyens visuels, les composants générant ladite opacification de la boisson comprenant des particules de dioxyde de titane.

2. Comprimé selon la revendication 1, **caractérisé en ce que** les composants générant l'opacification de la boisson sont constitués de particules de dioxyde de titane.

3. Comprimé selon l'une des revendications 1 et 2, **caractérisé en ce qu'**il comprend au moins 15 mg de particules de dioxyde de titane.

4. Comprimé selon une des revendications 1 à 3, **caractérisé en ce qu'**il comprend au moins un générateur d'effervescence ; avantageusement, ledit générateur est composé exclusivement d'un générateur de dioxyde de carbone, de préférence choisie parmi un carbonate ou un bicarbonate d'un métal alcalin, d'un métal alcalino-terreux ou d'un acide aminé, tel que le carbonate de calcium, le bicarbonate de sodium, le bicarbonate de potassium, le carbonate de potassium, le carbonate de L-lysine, le carbonate d'arginine, le sesquicarbonate de sodium et leurs mélanges ; avantageusement, le générateur de dioxyde de carbone est le carbonate de calcium.

5. Comprimé selon l'une quelconque des revendications 1 à 4, **caractérisé en ce qu'**il comprend en outre un excipient lipophile choisi parmi des stéarates, palmitate/stéarates et béhénates de glycéryle ; des huiles végétales hydrogénées et leurs dérivés ; des cires végétales et animales et leurs dérivés ; des huiles de ricin hydrogénées et leurs dérivés ; et des esters et alcools cétyliques ; de préférence, l'excipient lipophile est le béhénate de glycéryle.

6. Comprimé selon l'une quelconque des revendications précédentes, **caractérisé en ce que** l'agent délitant est choisi parmi le carboxyméthylamidon sodique, la carboxyméthylcellulose sodique réticulée et la polyvinylpyrrolidone réticulée, et plus avantageusement la polyvinylpyrrolidone réticulée.

7. Comprimé selon l'une quelconque des revendications précédentes, **caractérisé en ce que** l'agent soluble de type polyol est choisi parmi le mannitol, le xylitol, le sorbitol, le maltitol et leurs mélanges ; de préférence, l'agent soluble de type polyol est le mannitol.

8. Comprimé selon l'une quelconque des revendications précédentes, **caractérisé en ce que** la quantité de l'agent soluble de type polyol est compris entre 20 et 50 % en poids, de préférence entre 30 et 45 % en poids, par rapport au poids total du comprimé.

9. Comprimé selon l'une quelconque des revendications précédentes, **caractérisé en ce que** le zolpidem est enrobée avec un agent d'enrobage à base de polymère qui est soluble à pH 5 ou moins et qui est perméable à un pH supérieur à 5 ; en particulier, l'agent d'enrobage est un copolymère d'amino-méthacrylate.

10. Comprimé selon l'une quelconque des revendications précédentes, **caractérisé en ce qu'**il comprend en outre au moins un excipient hydrophile de préférence choisi parmi des dérivés de cellulose, hydroxyéthylcellulose, hydroxypropylcellulose, hydroxypropylméthylcellulose, carboxyméthylcellulose ou carboxyméthylcellulose sodique ; des gommes végétales et leurs dérivés ; des dérivés d'acide alginique ; des polyéthylène glycols et leurs dérivés ; des amidons et leurs dérivés ; des silices et leurs dérivés ; des polyméthacrylates ; et des copolymères d'acides acrylique et méthacrylique.

11. Comprimé selon l'une quelconque des revendications 1 à 10, **caractérisé en ce qu'**il comprend (1) une phase interne comprenant des granules enrobés à base de zolpidem et un agent d'enrobage à base de polymères qui est soluble à pH 5 ou moins et qui est perméable à un pH supérieur à 5, (2) une phase externe comprenant au moins un agent délitant, au moins un agent soluble de type polyol et des composants qui, si le comprimé est introduit dans une boisson aqueuse qui comprend facultativement de l'alcool, génèrent un moyen visuel correspondant à une opacification de la boisson et à au moins un autre moyen visuel choisi parmi l'effervescence du comprimé, le flottage du comprimé, la formation de particules insolubles et la combinaison de ces moyens visuels, les composants générant ladite opacification de la boisson comprenant des particules de dioxyde de titane.

12. Comprimé selon la revendication 11, **caractérisé en ce que** la phase externe comprend des particules de dioxyde de titane, un agent qui génère du dioxyde de carbone et au moins un excipient lipophile.

13. Comprimé selon la revendication 12, **caractérisé en ce que** la phase externe comprend des particules de dioxyde de titane, du carbonate de calcium et du béhénate de glycérol et est de préférence exempte de composé acide pharmaceutiquement acceptable.

14. Comprimé selon l'une quelconque des revendications précédentes, **caractérisé en ce qu'**il comprend de 1 à 5 % en poids de zolpidem, tel que, par exemple, 2,5, 5 ou 10 mg par comprimé, au moins 15 mg de particules de dioxyde de titane par comprimé, de 2 à 15 %, de préférence de 4 à 12 %, d'agent délitant et de 20 à 50 %, de préférence de 30 à 45 %, d'agent soluble de type polyol, et de préférence, il comprend en outre de 5 à 25 % en poids, de préférence de 10 à 20 % en poids, de générateur d'effervescence et de 0,05 à 15 %, de préférence de 0,1 à 6 %, d'excipients lipophiles, les pourcentages étant exprimés par rapport au poids total du comprimé.

15. Comprimé selon l'une quelconque des revendications précédentes pour utilisation orale dans un procédé pour le traitement de l'insomnie, en particulier l'insomnie occasionnelle, transitoire ou chronique.
